(19) Europäisches Patentamt
European Patent Office
Office européen des brevets

(11) Veröffentlichungsnummer: **0 164 668**
**B1**

(12) **EUROPÄISCHE PATENTSCHRIFT**

(45) Veröffentlichungstag der Patentschrift: **07.11.90**

(51) Int. Cl.[5]: **C 08 G 77/38,** A 61 K 7/06

(21) Anmeldenummer: **85106765.2**

(22) Anmeldetag: **01.06.85**

(54) **Betaingruppen enthaltende Siloxane, deren Herstellung und Verwendung in Haarpflegemitteln.**

(30) Priorität: **15.06.84 DE 3422268**

(43) Veröffentlichungstag der Anmeldung:
**18.12.85 Patentblatt 85/51**

(45) Bekanntmachung des Hinweises auf die Patenterteilung:
**07.11.90 Patentblatt 90/45**

(84) Benannte Vertragsstaaten:
**BE DE FR GB IT NL**

(56) Entgegenhaltungen:
**EP-A-0 017 121**
**EP-A-0 017 122**
**EP-A-0 166 122**

(73) Patentinhaber: **Th. Goldschmidt AG**
**Goldschmidtstrasse 100 Postfach 101461**
**D-4300 Essen 1 (DE)**

(72) Erfinder: **Kollmeier, Hans-Joachim, Dr.**
**Barkhorstrücken 27**
**D-4300 Essen (DE)**
Erfinder: **Langenhagen, Rolf-Dieter**
**Kampstrasse 5**
**D-4321 Hattingen-Niederwenigern (DE)**
Erfinder: **Hoffmann, Klaus**
**Ahornstrasse 47**
**D-4300 Essen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 164 668 B1**

**Beschreibung**

Die Erfindung betrifft neuartige Organopolysiloxane mit Betaingruppen. Sie betrifft ferner Verfahren zur Herstellung dieser Verbindungen. Sie betrifft schließlich die Verwendung dieser Verbindungen in kosmetischen Zubereitungen, insbesondere in Haarpflegemitteln.

Es ist bekannt, Organopolysiloxane zur Herstellung von Haarpflegemitteln zu verwenden. In "Chemie und Technologie der Silicone" von Walter Noll, Verlag Chemie, 2. Auflage, 1968, Seite 536, heißt es allerdings, daß die Aufgabe, die Frisur unabhängig von Feuchtigkeitseinflüssen zu erhalten, mit normalen Polydimethylsiloxanolen nicht zu lösen sei. Das Silicon müsse vielmehr mit Hilfe von funktionellen Gruppierungen auf dem Haar fixiert werden.

Aus der DE—AS 14 93 384 sind Organosiloxanverbindungen oder -verbindungsgemische der Formel

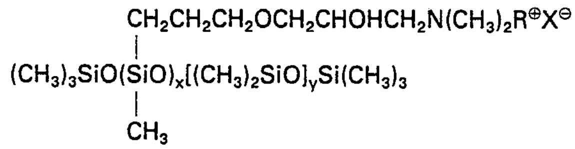

in der R für Wasserstoff oder $CH_3$ und X für Halogen steht und x = 1 bis 10 und y = 0 bis 8,5 sind, wobei y:x nicht größer als 8,5:1 ist, bekannt.

Diese Organosiloxane mit quaternären Ammoniumgruppen können dadurch hergestellt werden, daß man eine Epoxysiloxanverbindung der Formel

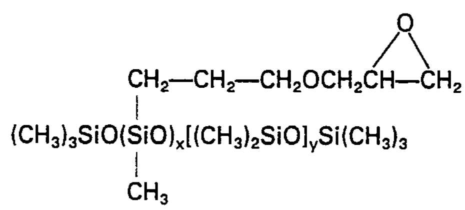

auf an sich bekannte Weise mit Dimethylamin umsetzt und die erhaltene Dimethylaminoorganosiloxanverbindung der Formel

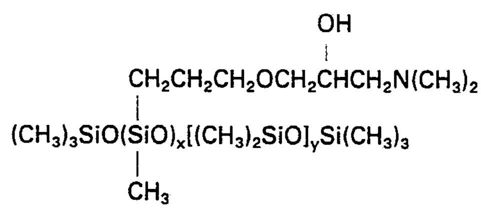

in an sich bekannter Weise mit einem Halogenwasserstoff oder mit einem Methylhalogenid in die quaternäre Ammoniumverbindung der vorgennten Formel überführt.

Die vorgenannten Organopolysiloxane mit quaternären Ammoniumgruppen können entsprechend der US—PS 4 185 087 für Haarpflegemittel verwendet werden. Dort ist ausgeführt, daß zwar einfache wäßrige Haarwaschmittel das Haar von Schmutz befreien und einen Überschuß an Fett entfernen können. Bei den meisten Haarwaschmitteln würde die Entfettung des Haares so gründlich vorgenommen, daß eine Schädigung des Haares zu beobachten ist. Die Haare würden sich nach der Wäsche elektrostatisch aufladen und deshalb schlecht kämmbar sein. Der Zusatz von Lanolinderivaten, Glykol, Fettsäureestern oder Proteinen würde zwar die Handhabbarkeit des Haares nach dem Waschen verbessern, gleichzeitig aber die Schaumbildung beeinträchtigen. Die Haare würden etwas klebrig und würden sich unnatürlich anfühlen. Die vorbeschriebenen Organopolysiloxane mit quaternären Ammoniumgruppen sollen nach den Angaben der US—PS 4 185 087 diese Nachteile beseitigen und die Kämmbarkeit der gewaschenen Haare, den Halt der Frisur, den Glanz der Haare verbessern.

Eine ähnliche Lehre ergibt sich aus den europäischen Patentschriften 0 017 121 und 0 017 122. Auch Hier sind Organopolysiloxane mit quaternären Ammoniumgruppen in Haarwasch- und Haarbehandlungsmitteln zur Verbesserung der Frisiertechnischen Eigenschaften der Haare beschrieben. Die Verbindungen entsprechen dabei der allgemeinen Formel

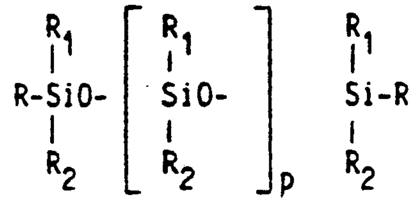

in der $R_1$ und $R_2$ einen Alkylrest mit 1 bis 4 Kohlenstoffatomen oder einen Arylrest, p die Zahlen 0 bis 50 und R die Reste

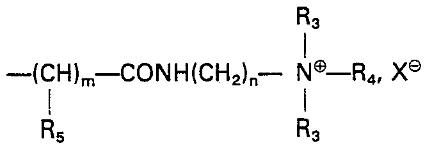

oder

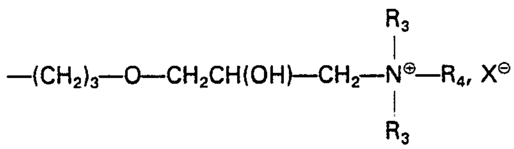

darstellen, wobei $R_3$ einen Alkyl- oder Hydroxy-alkylrest mit 1 bis 3 Kohlenstoffatomen, $R^4$ einen Rest gleich $R_3$ oder Aryl-$CH_2$— oder den Allylrest, $R_5$ Wasserstoff oder den Methylrest, $X^\ominus$ die Anionen $Cl^\ominus$, $Br^\ominus$, $J^\ominus$, $CH_3SO_4^\ominus$ oder $C_2H_5SO_4^\ominus$ und m die Zahlen 2 bis 10, n die Zahlen 2 bis 4 bedeuten.

Schließlich sei noch auf die veröffentlichte europäische Patentanmeldung 0 095 238 verwiesen, welche eine Zusammensetzung betrifft, die im wesentlichen aus folgenden Bestandteilen besteht:

A) einem Siloxan der allgemeinen Formel

$$R_aX_{3-a}Si(OSiX_2)_n(OSiX_bR_{2-b})_mOSiX_{3-a}R_a$$

wobei R nur aufgabenhaft als eine funktionelle Gruppe angegeben ist, welche die Haftung am Haar bewirkt, z.B. eine Amino-, Carbonsäure- oder quaternäre Ammoniumgruppe. X ist ein Wasserstoffrest oder eine Phenyl-, Hydroxyl- oder gesättigte Kohlenwasserstoffgruppe mit 1 bis 8 Kohlenstoffatomen. a hat einen Wert von 0 bis 3, b hat einen Wert von 0 bis 1 und n + m hat einen Wert von 1 bis 1999, wobei n einen Wert von 0 bis 2000 und m einen Wert von 1 bis 2000 hat,

B) einem Tensid,

C) einem Zusatz zur Verbesserung der Gefrier/Auftau-Stabilität und

D) Wasser.

Aus dem Stand der Technik ergibt sich somit, daß die Organopolysiloxane mit quaternären Ammoniumgruppen eine starke Substantivität auf dem Haar aufweisen und ihm gute Kämmbarkeit und Glanz verliehen. Ein Nachteil ist jedoch deren schlechte Verträglichkeit mit anionischen Komponenten, insbesondere mit anionischen Tensiden, in Haarpflegezubereitungen. Sie können darüber hinaus auch zu Irritationen der Haut, insbesondere der Schleimhaut und im Auge, führen. Dies ist aber gerade bei Haarwaschmitteln äußerst unerwünscht.

Der Erfindung liegt deshalb die Aufgabe zugrunde, Wirkstoffe für Haarkosmetika zu finden, welche zwar die guten Eigenschaften der Organopolysiloxane mit quaternären Ammoniumgruppen aufweisen, jedoch mit anionischen Wirkstoffen, insbesondere mit anionischen Tensiden, veträglich sind, wobei die Hautfreundlichkeit der neuen Siloxanderivate verbessert sein soll.

Überraschenderweise wurde gefunden, daß diese Eigenschaften bei Organopolysiloxanen zu finden sind, welche eine oder mehrere Betaingruppen aufweisen. Gegenstand der Erfindung sind somit zunächst Verbindungen der allgemeinen Formel

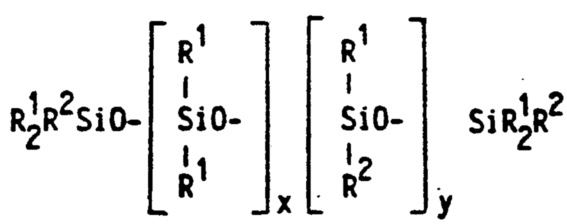

I

wobei

$R^1$ im Molekül gleich oder verschieden ist und einen Alkylrest mit 1 bis 18 Kohlenstoffatomen, einen Arylrest oder einen Polyoxyalkylenrest mit der Maßgabe bedeutet, daß mindestens 70% der Reste $R^1$ Methylreste sind,

$R^2$ gleich $R^1$ sein kann, mit der Maßgabe, daß mindestens ein Rest $R^2$ die Gruppe

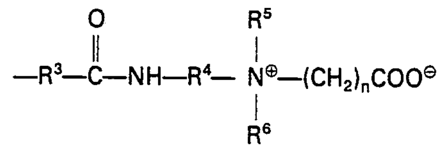

ist, in der

$R^3$ ein zweiwertiger Alkylenrest mit 2 bis 12 Kohlenstoffatomen,

$R^4$ ein zweiwertiger Alkylenrest mit 2 bis 6 Kohlenstoffatomen ist,

$R^5$, $R^6$ gleich oder verschieden sind und einen Alkylrest mit 1 bis 4 Kohlenstoffatomen oder einen Benzylrest bedeuten,

n 1, 2 oder 3 ist,

x einen Wert von 0 bis 200 und

y einen Wert von 1 bis 50 hat.

Aus der allgemeinen Formel I ist ersichtlich, daß die Betaingruppe(n) endständig oder seitenständig gebunden sein kann (können).

Mindestens 70% der Reste $R^1$ sind Methylreste. Besonders bevorzugt sind solche Polysiloxane, bei denen alle Reste $R^1$ Methylreste sind. Es können jedoch bis zu 30% der Reste $R^1$ Alkylreste mit 2 oder mehr, vorzugsweise aber 12 bis 18, Kohlenstoffatomen oder Arylreste sein. Beispiele solcher Alkylreste sind der Ethyl-, Propyl-, Isopropyl-, Butyl-, Hexyl-, Isooctyl-, Decyl-, Dodecyl- oder Stearylrest. Der Arylrest ist im allgemeinen ein Phenylrest. Sowohl die Alkylreste wie die Arylreste können substituiert sein.

Es ist ebenfalls möglich, daß bis zu 30% der Reste $R^1$ Polyoxyalkylenreste, und zwar insbesondere solche der allgemeinen Formel

$$—(CH_2)_3O(C_mH_{2m}O)_pQ$$

sind, wobei die Gruppe $—(C_mH_{2m}O)_p$ aus Ethylenoxid und Propylenoxid aufgebaut ist und m einen durchschnittlichen Wert von 2,0 bis 2,6 aufweist, p einen Wert von 1 bis 25 hat und Q ein Wasserstoff- oder ein Alkylrest mit 1 bis 4 Kohlenstoffatomen ist. Bevorzugt sind Verbindungen, in denen 3 bis 10% der Reste $R^1$ Polyoxyalkylenreste sind.

Mindestens ein Rest $R^2$ muß die Bedeutung der Gruppe

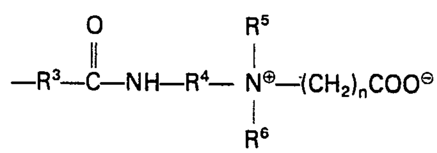

haben. $R^3$ und $R^4$ sind jeweils zweiwertige Alkylenreste. $R^3$ kann bis zu 12, $R^4$ bis zu 6 Kohlenstoffatome aufweisen. Vorzugsweise ist $R^3$ ein Alkylenrest mit 2 bis 10 Kohlenstoffatomen, $R^4$ ein Alkylenrest mit 2 bis 4 Kohlenstoffatomen. $R^5$ und $R^6$ sind dabei vorzugsweise Methylreste. Sie können jedoch auch Ethyl-, Propyl- oder Butylreste sein oder einen Benzylrest bedeuten. n hat einen Wert von 1, 2 oder 3, jedoch ist n = 1 bevorzugt.

x hat vorzugsweise einen Wert von 2 bis 100, insbesondere einen Wert von 5 bis 50.

y hat einen Wert von 1 bis 25, insbesondere einen Wert von 2 bis 10.

Aus der älteren europäischen Patentanmeldung 85105323.1—2107 (EP—A—0 166 122) sind Verbindungen der allgemeinen Formel

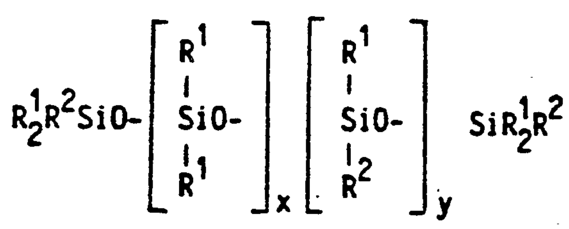

bekannt, wobei

$R^1$ im Molekül gleich oder verschieden ist und einen Alkylrest mit 1 bis 18 Kohlenstoffatomen, einen Arylrest oder einen Polyoxyalkylenrest mit der Maßgabe bedeutet, daß mindestens 70% der Reste $R^1$ Methylreste sind,

$R^2$ gleich $R^1$ sein kann, mit der Maßgabe, daß mindestens ein Rest $R^2$ die Gruppe

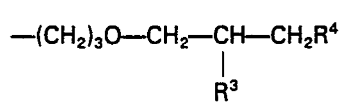

ist, in der $R^3$ und $R^4$ verschieden sind und ein Rest ein Hydroxylrest und der andere Rest die Gruppe

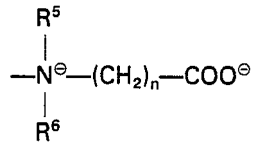

ist, in der $R^5$ und $R^6$ gleich oder verschieden sind und einen Alkyrest mit 1 bis 4 Kohlenstoffatomen oder einen Benzylrest bedeuten, und n = 1, 2 oder 3 ist,

x einen Wert von 0 bis 200 und

y einen Wert von 1 bis 50 hat.

Diese Verbindungen unterscheiden sich von den hier beanspruchten Verbindungen durch das Brückenglied, welches die Betaingruppe mit dem Polysiloxangerüst verbindet. Die Verbindungen unterscheiden sich deshalb auch in ihrem anwendungstechnischen Verhalten.

Beispiele erfindungsgemäßer Organopolysiloxane mit Betaingruppen sind

a) 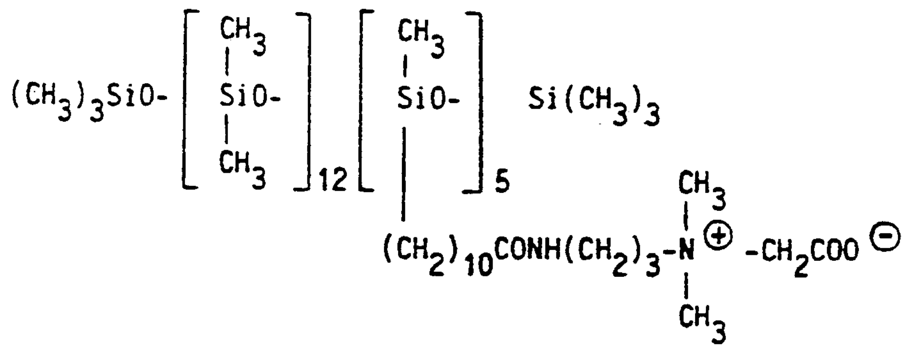

b) 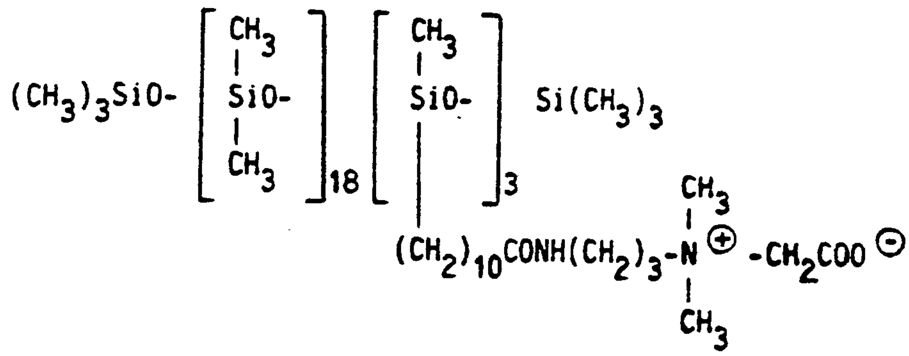

c) 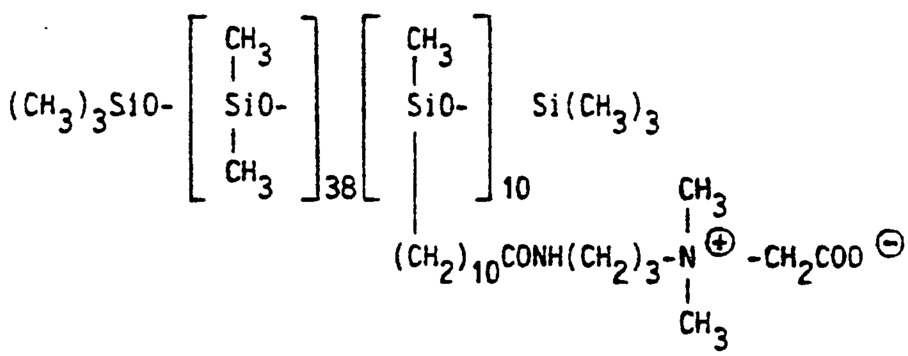

d) 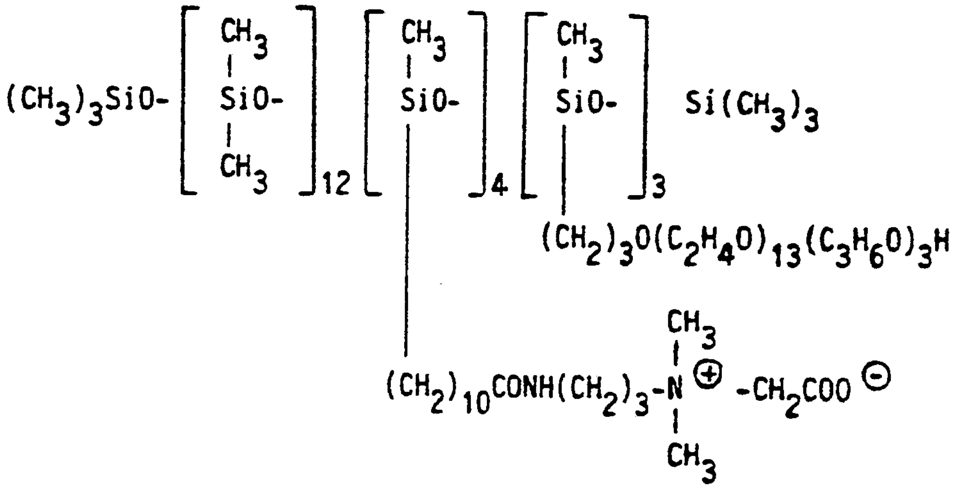

e) 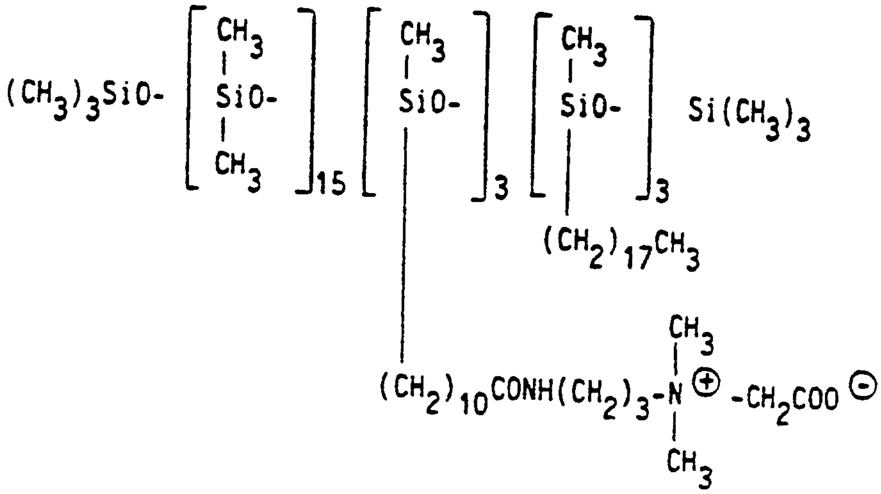

Die erfindungsgemäßen Verbindungen sind im allgemeinen viskose bis hochviskose, öbige bis pastenartige und farblose bis gelb gefärbte Produkte. Die Löslichkeit der erfindungsgemäßen Verbindungen wird im wesentlichen durch das Verhältnis der Anzahl der Betaingruppen zu der Anzahl der Siloxyeinheiten ($R^1_2SiO$) sowie der Art der Reste $R^1$ bestimmt. Höhere Gehalte an Betaingruppen und/oder die Anwesenheit von Polyoxyalkylenresten mit überwiegendem Anteil von Oxyethyleneinheiten ergeben Produkte, die in Wasser und niedrigen Alkoholen oder Glykolen löslich sind. Durch den Einbau von Alkylresten mit 12 bis 18 Kohlenstoffatomen können demgegenüber ölverträgliche bzw. öldispergierbare Produkte erhalten werden. Für die Anwendung in Haarpflegemitteln sind im allgemeinen Produkte bevorzugt, die in Wasser oder Glykolen löslich sind.

Ein weiterer Gegenstand der Erfindung besteht in der Herstellung der erfindungsgemäßen Verbindungen. Das Verfahren zur Herstellung der erfindungsgemäßen Organopolysiloxane mit Betaingruppen besteht darin, daß man Verbindungen der allgemeinen Formel

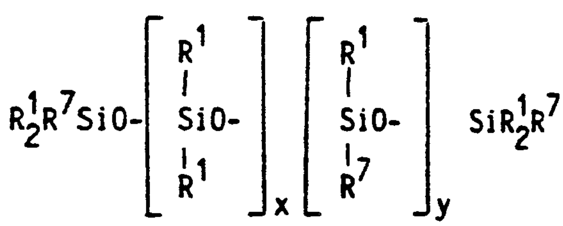 II

wobei

$R^7$ gleich $R^1$ sein kann, mit der Maßgabe, daß mindestens ein Rest $R^7$ ein Wasserstoffrest ist, zunächst mit, in bezug auf SiH-Gruppen, äquimolaren Mengen Verbindungen der allgemeinen Formel

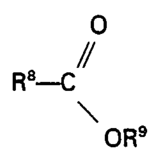 III

wobei

$R^8$ ein Alkenrest mit endständiger Doppelbindung und 2 bis 12 Kohlenstoffatomen oder ein Cycloalkylenrest mit 6 bis 12 Kohlenstoffatomen,

$R^9$ ein Wasserstoff- oder Alkylrest mit 1 bis 4 Kohlenstoffatomen ist,

in Gegenwart von Platin- oder Palladiumkatalysatoren in an sich bekannter Weise umsetzt und das erhaltene Produkt sodann mit Verbindungen der allgemeinen Formel

$$\begin{array}{r} R^5 \\ | \\ H_2N{-}R^4{-}N \\ | \\ R^6 \end{array} \qquad IV$$

in an sich bekannter Weise umsetzt und das erhaltene Produkt schließlich mit äquimolaren Mengen Verbindungen der allgemeinen Formel

$$X{-}(CH_2)_nCOOY \qquad V$$

wobei

X ein Chlor- oder Bromrest und

Y ein Alkalirest ist,

in an sich bekannter Weise quaterniert.

Zur Katalyse der Addition der eine olefinische Doppelbindung enthaltenden Verbindungen III an die Wasserstoffsiloxane II werden Platin- oder Palladiumverbindungen eingesetzt. Ein geeigneter Katalysator ist $H_2PtCl_6 . 6 H_2O$. Weitere besonders geeignete Katalysatoren sind aus der DE-PS 31 33 869 bekannt. Sie werden in Mengen von $10^{-2}$ bis $10^{-8}$ Mol je Mol SiH-Gruppen im Siloxan verwendet. Bevorzugte Anlagerungstemperaturen sind 40 bis 160°C, insbesondere 50 bis 120°C. Bei Verwendung eines inerten Lösungsmittels bestimmt dessen Siedepunkt die obere Temperaturgrenze.

Das erhaltene Reaktionsprodukt wird nun durch Umsetzung mit den Verbindungen IV in das Säureamid überführt und schließlich mit den Verbindungen V quaterniert. Auch diese Reaktionen laufen in an sich bekannter Weise, vorzugsweise bei Temperaturen von 40 bis 160°C ab. Als inerte Lösungsmittel können Alkohole, Glykole oder Wasser verwendet werden. Das Endprodukt kann durch Filtration vom Alkalisalz befreit werden.

Die erfindungsgemäßen Verbindungen zeigen die gewünschte Eigenschaftskombination. Der Vergleich mit nach dem Stand der Technik verwendeten ähnlichen Produkten kann der folgenden Tabelle entnommen werden.

TABELLE

| Wirkstoff | Substantivität von Haar | Kämmbarkeit und Glanz der Haare | Hautirritation | Verträglichkeit mit anionischen Wirkstoffen |
|---|---|---|---|---|
| Siloxan mit Polyethergruppen | Schwach | schwach | keine | gut |
| Siloxan mit anionischen Gruppen | keine | keine | schwach | gut |
| Siloxan mit Aminogruppen | mittel | mittel | mittel | gut |
| Siloxan mit quaternären Aminogruppen | stark | gut | schwach | schlecht |
| Siloxan mit Betaingruppen | mittel | gut | keine | gut |
| siloxanfreies Betain | mittel | keine | keine | gut |

Aus der Tabelle ist ersichtlich, daß die Organopolysiloxane mit Betaingruppen die gewünschte Eigenschaftskombination aufweisen, die keine der übrigen Verbindungen zeigt.

Es ist deshalb ein weiterer Gegenstand der Erfindung, die erfindungsgemäßen Verbindungen in kosmetischen Zubereitungen, insbesondere in Zubereitungen zur Pflege der Haare, zu verwenden. Haarkosmetika können dabei Haarwaschmittel oder Haarpflegemittel sein, je nachdem ob der Reinigungseffekt oder der Pflegeeffekt und der Effekt der besseren Kämmbarkeit im Vordergrund steht. Haarwaschmittel, denen die erfindungsgemäßen Organopolysiloxane mit Betaingruppen in Mengen von 0,1 bis 10 Gew.-% zugesetzt sind und die bis zu 30 Gew.-% waschaktiver Substanzen neben Wasser und gegebenenfalls anderen Zusatzstoffen enthalten, bewirken, daß die gewaschenen Haare Fülle und angenehmen Griff aufweisen, den gewünschten Glanz zeigen und leicht kämmbar sind. Eine elektrostatische Aufladung der Haare ist praktische nicht zu beobachten. In Haarpflegemitteln, wie Haarwässern, bewirken die erfindungsgemäßen Verbindungen in Mengen von 0,1 bis 5% bereits eine wesentliche Verbesserung der Kämmbarkeit der Haare und bilden Fülle und Glanz aus.

Den Haarpflegemitteln können übliche Zusatzmittel, wie Lösungsmittel, Verdickungsmittel, Parfüm, Konservierungsmittel, Komplexbildner, Schaumstabilisierungsmittel, Trübungsmittel, Perlglanzmittel oder andere übliche Zusatzstoffe, wie Farbstoffe, zugegeben werden.

Im folgenden sind Richtrezepturen für Haarpflegemittel angegeben:

Mittel für eine Cremekurspülung

| | |
|---|---|
| Cetylalkohol | 6 Gew.-Teile |
| Teginacid® H* | 6 Gew.-Teile |
| Glycerin | 3 Gew.-Teile |
| Betain-Siloxan (gemäß Beispiel 1) | 2 Gew.-Teile |
| Wasser | 83 Gew.-Teile |

*Teginacid ist ein eingetragenes Warenzeichen der Th. Goldschmidt AF. Unter der Produktbezeichnung Teginacid H wird eine Mischung von Glycerinmono-distearat mit einem geringen Anteil von Polyglykolfettalkoholethern vertrieben.

Konditioniershampoo

| | |
|---|---|
| a) Natriumlaurylethersulfat | 3 Gew.-Teile |
| Ammoniumalkylethersulfat | 6 Gew.-Teile |
| Tagat® KL 141* | 5 Gew.-Teile |
| Betain-Siloxan (gemäß Beispiel 1) | 2 Gew.-Teile |
| Wasser | 84 Gew.-Teile |

*Tagat ist ein eingetragenes Warenzeichen der Th. Goldschmidt AG. Unter der Produktbezeichnung Tagat KL 141 wird ein Polyoxyethylenpropylenglykoldioleat vertrieben.

| | |
|---|---|
| b) Kokosfettsäurediethanolamid | 0,5 Gew.-Teile |
| Natriumlaurylethersulfat | 30 Gew.-Teile |
| Kochsalz | 1,5 Gew.-Teile |
| Tego®-Betain L 7* (Alkylamidobetain) | 8 Gew.-Teile |
| Betain-Siloxan (gemäß Beispiel 1) | 2 Gew.-Teile |
| Wasser | 58 Gew.-Teile |

*Tego ist ein eingetragenes Warenzeichen der Th. Goldschmidt AG. Unter der Produktbezeichnung Tego-Betain L 7 wird ein Cocamidopropylbetain (1-Alkylamino-3-dimethylammonium-propan-3-carboxymethylbetain) vertrieben.

Die erfindungsgemäßen Verbindungen können auch Hauptflegemitteln zugegeben werden. Als Bedstandteil von Seifen oder Hautcremes bilden sie auf der Haut einen feinen, nicht hautreizenden, nicht fettenden Film aus. Im Gegensatz zu niedrigviskosen, insbesondere cyclischen, Dimethylsiloxanen verdunsten sie nicht auf der Haut und ergeben daher einen beständigen Schutz.

Dabei kann eine flüssige Seife folgendermaßen zusammengestzt sein:

| | |
|---|---|
| Tagat® 0 2* | 1 Gew.-Teil |
| Kokosfettsäurediethanolamid | 0,5 Gew.-Teile |
| Natriumlaurylethersulfat | 30 Gew.-Teile |
| Tego®-Betain L 7* (Alkylamidobetain) | 7 Gew.-Teile |
| Kochsalz | 2 Gew.-Teile |
| Betain-Siloxan (gemäß Beispiel 3) | 3 Gew.-Teile |
| Wasser | 56.5 Gew.-Teile |

*Tagat ist ein eingetragenes Warenzeichen der Th. Goldschmidt AG. Unter der Produktbezeichnung Tagat 0 2 wird ein Polyoxyethylenglycerin-monooleat vertrieben.

In den folgenden Beispielen wird das erfindungsgemäße Verfahren näher erläutert. Es werden ferner anwendungstechnische Überprüfungen im Vergleich mit Produkten des Standes der Technik gezeigt.

Beispiel 1

Herstellung des Betaingruppen enthaltenden Siloxans der Formel

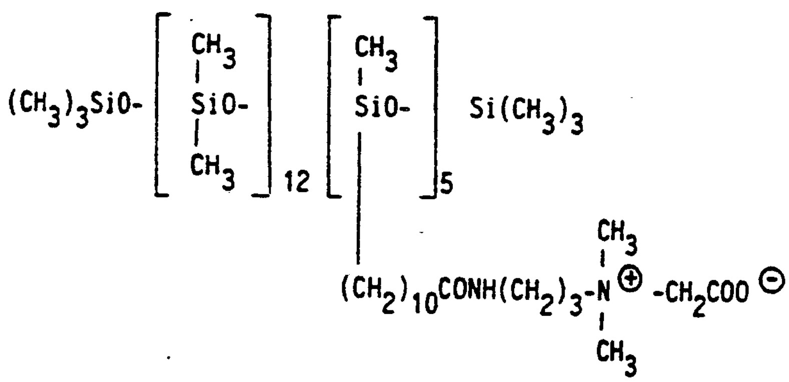

a) Zunächst werden in einem Kolben, der mit Rührer, Thermometer, Gaseinleitung und Rückflußkühler versehen ist, 123,9 g Undecylensäuremethylester und 0,15 g einer 10 %igen Lösung von $H_2PtCl_6 . 6 H_2O$ in i-Propanol bei 120°C vorgelegt. Zu diesem Gemisch werden in 20 Min. 169,1 g eines Siloxans mit der durchschnittlichen Formel

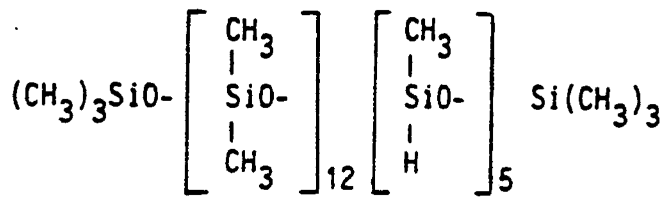

zugetropft. Die Reaktion wird unter Abdeckung mit Stickstoff durchgeführt. Nach 4,5 Std. ergibt sich as der Analyse des noch vorhandenen aktiven Wasserstoffs ein Umsatz von 92%. Der Ansatz wird mit 5 g $NaHCO_3$ versetzt, 30 Min. gerührt und filtriert. Man erhält 280 g eines Siloxans der durchschnittlichen Formel

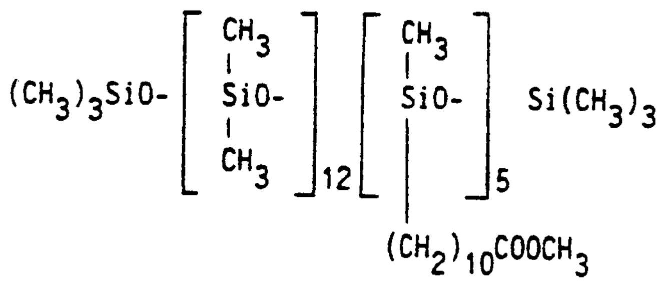

A

b) In einem Kolben, der mit Rührer, Thermometer, Gaseinleitung und Vorrichtung zur Destillation versehen ist, werden 234,5 g (= 0,1 Mol) des erhaltenen Siloxans A und 100 g 3-Dimethylamino-1-propylamin (entspechend etwa einem 100 %igen Überschuß) gemischt. Unter Einleiten eines leichten Stickstoffstroms wird das Gemisch auf 130°C erhitzt und 1 Std. gerührt. Die Temperatur wird dann auf 160°C erhöht und 2 Std. beibehalten. Dabei fallen 17,5 g Destillat an. Der Ansatz wird abgekühlt und das überschüssige Amin durch Destillation im Wasserstrahlvakuum bis zu einer Badtemperatur von 140°C entfernt. Als Rückstand erhält man eine gelbbraune, klare, viskose Flüssigkeit. Die Analyse ergibt einen Amin-Stickstoffgehalt von 2,55% (theoretisch: 2,60%). Das erhaltene Siloxan entspricht der durchschnittlichen Formel

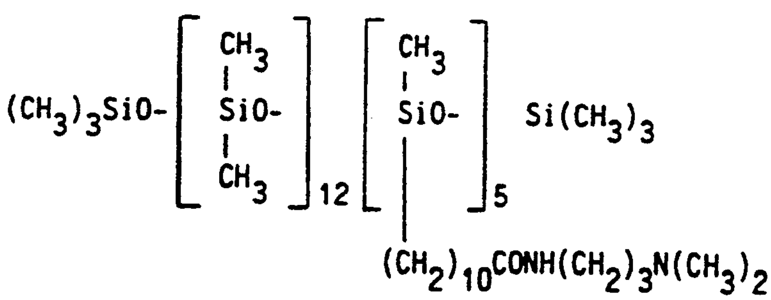

B

c) In einem Kolben, der mit Rührer, Thermometer und Rückflußkühler versehen ist, werden 164,8 g (= 0,3 Mol Aminogruppen) des Siloxans der Formel B und 34,9 g (= 0,3 Mol) $ClCH_2COONa$ in 150 g Wasser und 50 g Isopropanol gegeben und 5 Std. bei 85°C geührt. Nach etwa 1 Std. wird der Ansatz klar. Nach der Reaktion lassen sich in der erhaltenen Lösung 2,6% ionogenes Chlor bestimmen; dies entspricht einem Umsatz von 97,7%. Anschließend wird das Lösungsmittel in einem Rotationsverdampfer im Wasserstrahlvakuum bis zu einer Badtemperatur von 70°C abdestilliert. Es verbleibt ein trüber Rückstand, der bei Raumtemperatur fast fest ist. Um das entstandene Natriumchlorid zu entfernen, wird der Rückstand in i-Propanol aufgenommen und filtriert. Anschließend wird erneut im Rotationsverdampfer eingeengt. Man erhält ein klares, hellbraunes Produkt, das bei Raumtemperatur kaum fließfähig ist. Die Analyse ergibt einen Stickstoffgehalt, gebunden in der Betaingruppe, von 2,0% (theoretisch: 2,31%).

Beispiel 2

Herstellung des Betaingruppen enthaltenden Siloxans der Formel

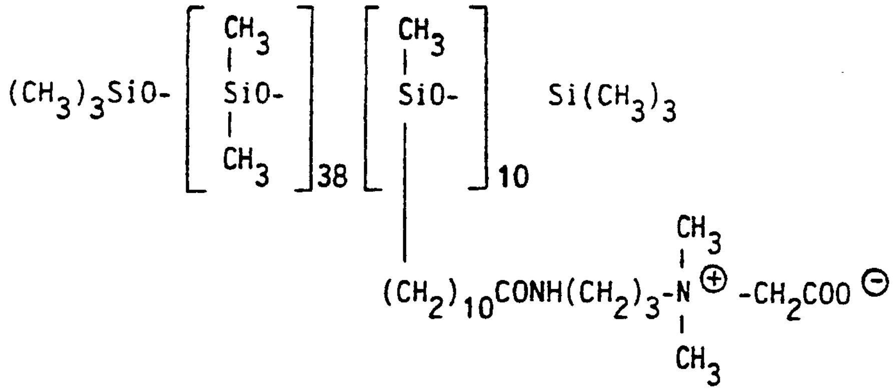

a) Zunächst wird analog Beispiel 1, Stufe a), an ein Siloxan der Formel

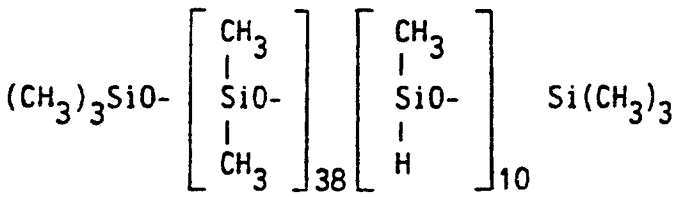

Undecylensäuremethylester angelagert, wobei ein Siloxan der Formel

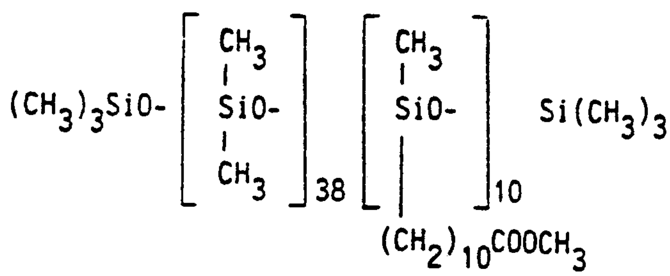

C

erhalten wird.

b) 278.2 g (= 0.05 Mol) des Siloxans der Formel C werden, wie in Beispiel 1, Stufe b), beschrieben, mit 100 g (entsprechend etwa einem 100 %igen Überschuß) 3-Dimethylamino-1-propylamin umgesetzt. Bei der Reaktion fallen 15,5 g Destillat an. Das erhaltene Produkt ist eine gelbbraune, klare, viskose Flüssigkeit und enthält 2,2% Amin-Stickstoff (theoretisch: 2,24%). Das Produkt entspricht der Formel

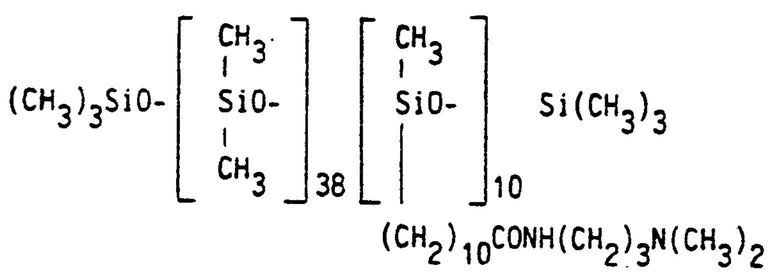

D

c) In einem mit Rührer, Thermometer und Rückflußkühler versehenen Kolben werden 254,7 g (= 0,4 Mol Aminogruppen) des Siloxans der Formel D, eine Lösung von 46,6 g (= 0,4 Mol) $ClCH_2COONa$ in 254,5 g Wasser sowie 370,5 g 1,2-Propylenglykol gegeben und 5 Std. bei 100°C gerührt. Der Ansatz wird nach etwa 1 Std. klar. Nach der Reaktion lassen sich in der erhaltenen klaren, gelben Lösung 1,45% ionogen gebundenes Chlor bestimmen. Dies entspricht einem Umsatz von 94,8%. In der Lösung beträgt der Gehalt an in der Betaingruppe gebundenem Stickstoff 0,56% (theoretisch: 0,60%).

Die erhaltene Lösung enthält 30 Gew.-% des erfindungsgemäßen Siloxans, 2,5 Gew.-% Natriumchlorid, 27,5 Gew.-% Wasser und 40 Gew.-% 1,2-Propylenglykol.

Beispiel 3

Herstellung des Betaingruppen enthaltenden Siloxans der Formel

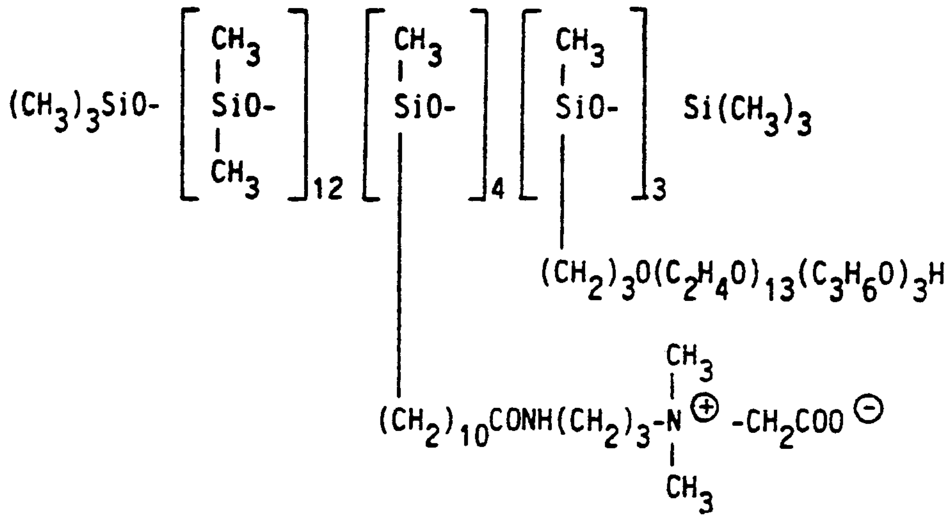

a) Zunächst wird ein Gemisch aus 99,2 g Undecylensäuremethylester, 301,5 g Polyoxyalkylenmonool der Formel

$$CH_2{=}CH{-}CH_2O{-}(C_2H_4O{-})_{13}(C_3H_6O{-})_3H$$

und 0,3 g einer 10 %igen Lösung von $H_2PtCl_6 . 6\,H_2O$ in i-Propanol mit 184,1 g eines Siloxans der durchschnittlichen Formel

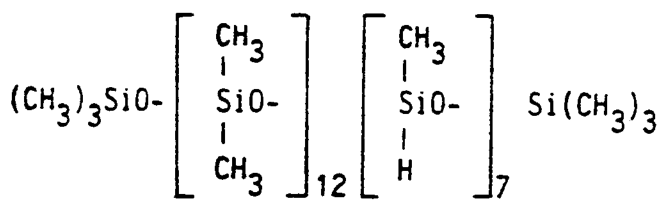

wie in Beispiel 1, Stufe a), beschrieben, umgesetzt. Der Umsatz beträgt 89,5%. Man erhält 564 g eines Siloxans der durchschnittlichen Formel

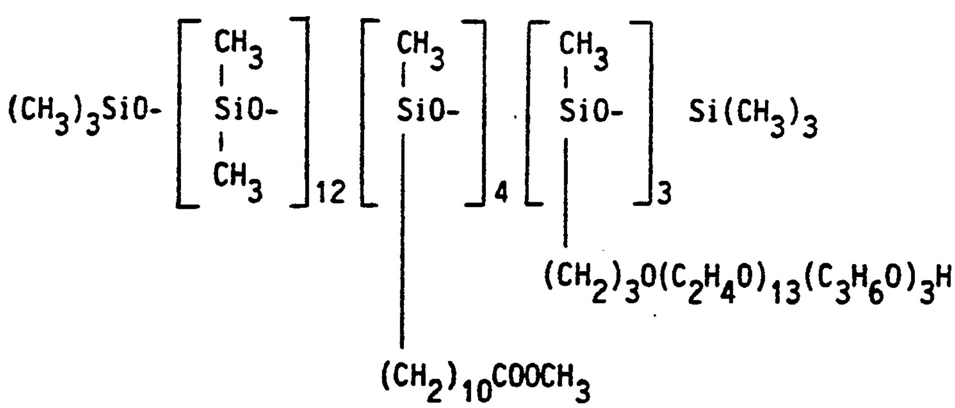
E

b) Analog Beispiel 1, Stufe b), werden 467,8 g (= 0,1 Mol) des Siloxans der Formel E mit 82 g (entsprechend einem Überschuß von etwa 100%) 3-Dimethylamino-1-propylamin umgesetzt. Dabei fallen 12 g Destillat an. Das erhaltene Siloxan ist eine gelbbraune, klare, viskose Flüssigkeit. Der Gehalt an Amin-Stickstoff beträgt 1,08% (theoretisch: 1,13%). es entspricht der Formel

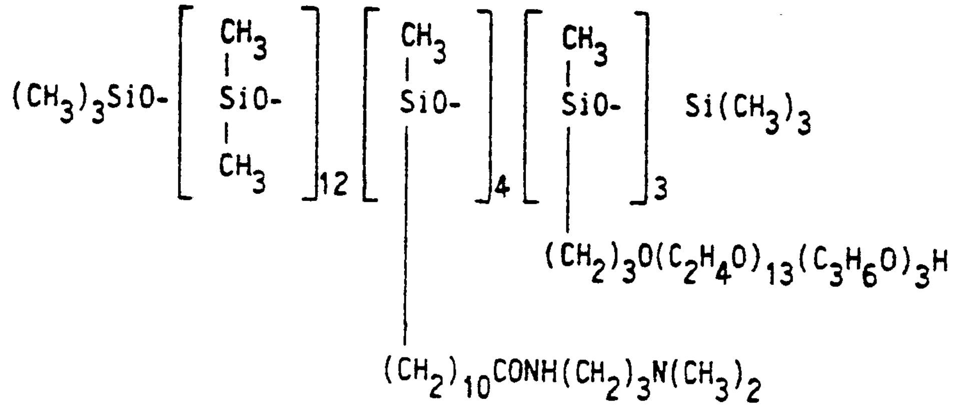

F

c) In einem Kolben, der mit Rührer, Thermometer und Rückflußkühler versehen ist, werden 259,4 g (= 0,2 Mol Aminogruppen) des Siloxans der Formel F und eine Lösung von 23,3 g (= 0,2 Mol) $ClCH_2COONa$ in 259,3 g Wasser sowie 135,5 g 1,2-Propylenglykol gegeben und 5 Std. bei 100°C gerührt. Nach etwa ½ Std. wird der Ansatz klar. Nach der Reaktion lassen sich in der erhaltenen klaren, gelben Lösung 1,0% ionogen gebundenes Chlor bestimmen, entsprechend einem Umsatz von 95,2%. In der Lösung beträgt der Gehalt an in der Betaingruppe gebundenem Stickstoff 0,38% (theoretisch: 0,41%).

Die erhaltene Lösung enthält 40 Gew.-% des erfindungsgemäßen Siloxans, 1,7 Gew.-% Natriumchlorid, 38,3 Gew.-% Wasser und 20 Gew.-% 1,2-Propylenglykol.

Beispiel 4

Überprüfung der erfindungsgemäßen Verbindungen in Haarpflegemitteln

Ein Konditioniershampoo folgender Zusammensetzung

| | |
|---|---|
| Natriumlaurylethersulfat | 3 Gew.-Teile |
| Ammoniumalkylethersulfat | 6 Gew.-Teile |
| Tagat® KL 141* | 5 Gew.-Teile |
| Betain-Siloxan (gemäß Beispiel 2) | 3 Gew.-Teile |
| Wasser | 83 Gew.-Teile |

*Tagat ist ein eingetragenes Warenzeichen der Th. Goldschmidt AG. Unter der Produktbezeichnung Tagat KL 141 wird ein Polyoxythylenpropylenglykoldioleat vertrieben

wird in sein Wirkungsweise mit einer Shampoo-Formulierung verglichen, in der das erfindungsgemäße Betain-Siloxan durch eine kationaktive Organossiloxanverbindung gemäß DE—AS 14 93 384 mit x = 15, y = 5 und X = $Cl^{\ominus}$ ersetzt wird.

Das Präparat mit dem Betain-Siloxan ist klar, während das kationaktive Siloxan zur Eintrübung führt.

In der praktischen Anwendung beim halbseitigen Vergleichstest auf menschlichem Haar ergibt sich folgende Beurteilung:

Das erfindungsgemäße Betain-Siloxan ist in der Schaumbildung, der Cremigkeit des Schaumes, der Trockenkämmbarkeit, der antielektrostatischen Wirkung und der Fülle des Haares überlegen. Lediglich in der Naßkämmbarkeit ergibt das kationaktive Siloxan ein etwas besseres Resultat. Dies ist jedoch mit einer stärkeren Beschwerung und damit einer geringeren Fülle des Haares verbunden.

**Patentansprüche**

1. Verbindungen der allgemeinen Formel

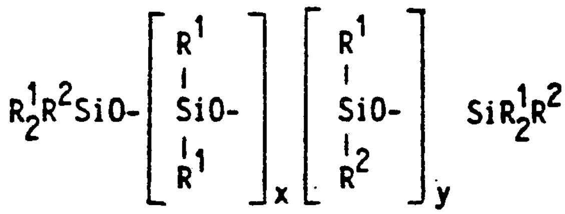

wobei

$R^1$ im Molekül gleich oder verschieden ist und einen Alkylrest mit 1 bis 18 Kohlenstoffatomen, einen Arylrest oder einen Polyoxyalkylenrest mit der Maßgabe bedeutet, daß mindestens 70% der Reste $R^1$ Methylreste sind,

$R^2$ gleich $R^1$ sein kann, mit der Maßgabe, daß mindestens ein Rest $R^2$ die Gruppe

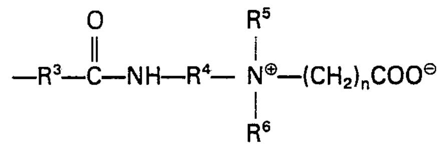

ist, in der

$R^3$ ein zweiwertiger Alkylenrest mit 2 bis 12 Kohlenstoffatomen,

$R^4$ ein zweiwertiger Alkylenrest mit 2 bis 6 Kohlenstoffatomen ist,

$R^5$, $R^6$ gleich oder verschieden sind und einen Alkylrest mit 1 bis 4 Kohlenstoffatomen oder einen Benzylrest bedeuten,

n 1, 2 oder 3 ist,

x einen Wert von 0 bis 200 und

y einen Wert von 1 bis 50 hat.

2. Verfahren zur Herstellung der Verbindungen des Anspruchs 1, dadurch gekennzeichnet, daß man Verbindungen der Allgemeinen Formel

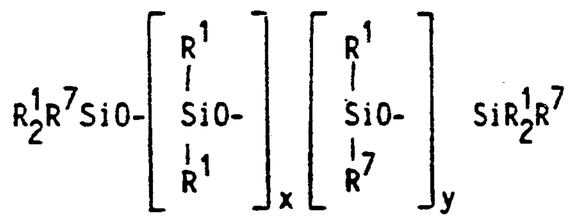

wobei

$R^7$ gleich $R^1$ sein kann, mit der Maßgabe, daß mindestens ein Rest $R^7$ ein Wasserstoffrest ist, zunächst mit, in bezug auf SiH-Gruppen, äquimolaren Mengen Verbindungen der allgemeinen Formel

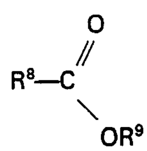

wobei

$R^8$ ein Alkenrest mit endständiger Doppelbindung und 2 bis 12 Kohlenstoffatomen oder ein Cycloalkylenrest mit 6 bis 12 Kohlenstoffatomen,

$R^9$ ein Wasserstoff- oder Alkylrest mit 1 bis 4 Kohlenstoffatomen ist,

in Gegenwart von Platin- oder Palladiumkatalysatoren in an sich bekannter Weise umsetzt und das erhaltene Produkt sodann mit Verbindungen der allgemeinen Formel

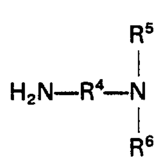

in an sich bekannter Weise umsetzt und das erhaltene Produkt schließlich mit äquimolaren Mengen Verbindungen der allgemeinen Formel

$$X—(CH_2)_nCOOY$$

wobei

X ein Chlor- oder Bromrest und

Y ein Alkalirest ist,

in an sich bekannter Weise quaterniert.

3. Verfahren nach Anspruch 2, dadurch gekennzeichnet, daß man die Umsetzungen in einem inerten Lösungsmittel und-oder bei Temperaturen von 40 bis 160°C oder bis zum Siedepunkt des verwendeten Lösungsmittels durchführt.

4. Verwendung der Verbindungen des Anspruch 1 in kosmetischen Zubereitungen, insbesondere in Zubereitungen zur Pflage der Haare.

**Revendications**

1. Composés répondant à la formule générale

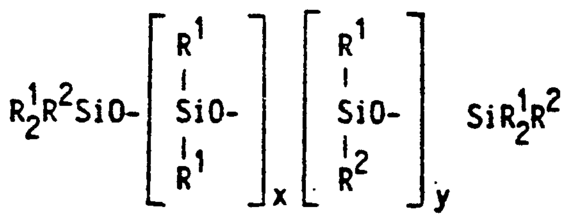

dans laquelle

$R^1$ est identique ou différent dans la molécule et représente un radical alkyle contenant de 1 à 18 atomes de carbone, un radical aryle ou un radical polyoxyalkylène à condition qu'au moins 70% des radicaux $R^1$ soient des radicaux méthyle.

$R^2$ peut ête identique à $R^1$, à condition qu'au moins un radical $R^2$ soit le groupe

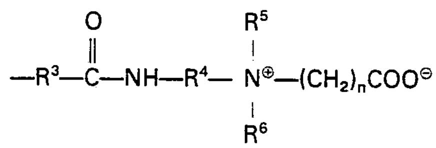

dans lequel

$R^3$ représente un radical alkylène bivalent contenant de 2 à 12 atomes de carbone,

$R^4$ représente un radical alkylène bivalent contenant de 2 à 6 atomes de carbone,

$R^5$, $R^6$ sont identiques ou différents et représentent un radical alkyle contenant de 1 à 4 atomes de carbone ou un radical benzyle,

n est égal à 1, 2 ou 3,

x a une valeur comprise entre 0 et 200 et

y a une valeur comprise entre 1 et 50.

2. Procédé de préparation des composés selon la revendication 1, caractérisé en ce qu'on fait d'abord réagir d'une manière connue des composés répondant à la formule générale

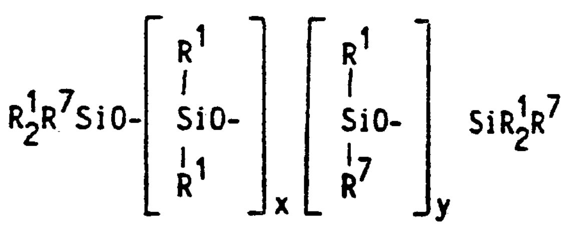

(dans laquelle $R^7$ peut être identique à $R^1$, à condition qu'au moins un radical $R^7$ soit un radical hydrogène) avec des quantités équimolaires, par rapport aux groupes SiH, de composés répondant à la formule générale

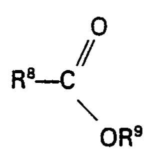

(dans laquelle $R^8$ représente un radical alcène contenant une double liaison en fin de chaîne et de 2 à 12 atomes de carbone ou un radical cycloalkylène contenant de 6 à 12 atomes de carbone et $R^9$ représente un

radical hydrogène ou un radical alkyle contenant de 1 à 4 atomes de carbone), en présence de catalyseurs de platine ou de palladium et qu'on fait ensuite réagir, d'une manière connue, le produit obtenu avec des composés répondant à la formule générale

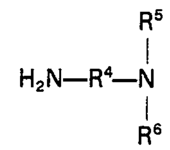

et qu'on quaternise enfin le produit obtenu au moyen de quantités équimolaires de composés répondant à la formule générale

$$X—(CH_2)_nCOOY$$

(dans laquelle X représente un radical de chlore ou de brome et Y représente un radical alcalin).

3. Procédé selon la revendication 2, caractérisé en ce qu'on réalise les réactions dans un solvant inerte et/ou à des températures allant de 40 à 160°C ou jusqu'au point d'ébullition du solvant mis en oeuvre.

4. Mise en oeuvre des composés de la revendication 1 dans des préparations cosmétiques, en particulier dans des préparations pour le soin des cheveux.

**Claims**

1. Compounds of the general formula

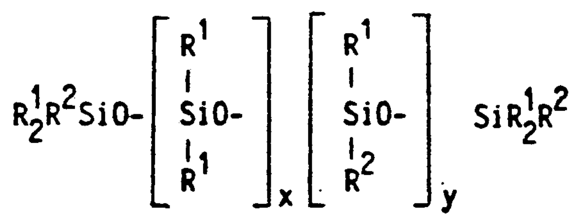

in which

$R^1$ in the molecule, is identical or different and denotes an alkyl radical having 1 to 8 carbon atoms, an aryl radical or a polyoxyalkylene radical with the proviso that at least 70% of the $R^1$ radicals are methyl radicals,

$R^2$ may be equal to $R^1$, with the proviso that at least one radical $R^2$ is the group

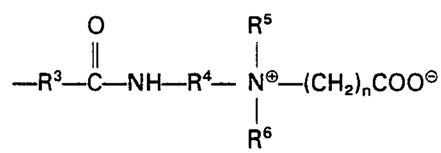

in which

$R^3$ denotes a divalent alkylene radical having 2 to 12 carbon atoms,

$R^4$ denotes a divalent alkylene radical having 2 to 6 carbon atoms,

$R^5$ and $R^6$ are identical or different and denote an alkyl radical having 1 to 4 carbon atoms or a benzyl radical,

n is 1, 2 or 3,

x has a value of 0 to 200 and

y has a value of 1 to 50.

2. Process for the preparation of the compounds of Claim 1, characterized in that compounds of the general formula

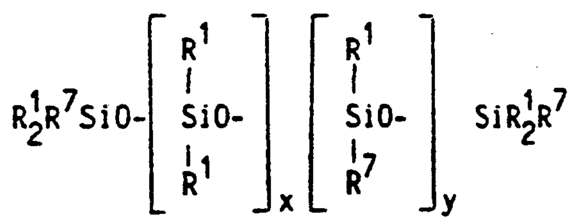

in which

$R^7$ may be equal to $R^1$, with the proviso that at least one radicaal $R^7$ is a hydrogen radical,

are first reacted in a manner known per se with, in respect of SiH groups, equimolar amounts of compounds of the general formula

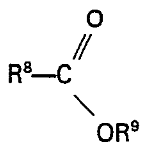

in which

$R^8$ is an alkene radical having a terminal double bond and 2 to 12 carbon atoms or a cycloalkylene radical having 6 to 12 carbon atoms and

$R^9$ is a hydrogen or alkyl radical having 1 to 4 carbon atoms,

in the presence of platinum or palladium catalysts and the product obtained is then reacted in a manner known per se with compounds of the general formula

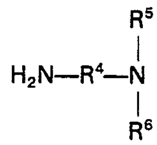

and the product obtained is finally quaternized in a manner known per se with equimolar amounts of compounds of the general formula

$$X—(CH_2)_nCOOY$$

in which

X is a chlorine or bromine radical and

Y is an alkali metal radical.

3. Process according to Claim 2, characterized in that the reactions are carried out in an inert solvent and/or at temperatures of 40 to 160°C or up to the boiling point of the solvent used.

4. Use of the compounds of Claim 1 in cosmetic preparations, in particular in preparations for care of the hair.